# EUROPEAN PATENT APPLICATION

(11) **EP 4 844 653 A1**
(43) Date of publication of application: **25.11.2026**
(21) Application number: 26177673.6
(22) Date of filing: 08.05.2026
(51) Int. Cl.: G06Q 10/087, G16H 40/20

(54) **PRODUCT EXPIRATION MANAGEMENT AND RISK MITIGATION SYSTEM**

(30) Priority: 14.05.2025 US 202519207443
(71) Applicant: DeRoyal Industries, Inc., Powell, TN 37849 (US)
(72) Inventor: DEBUSK, Brian C., Knoxville, TN 37922 (US); SMITH, Joe L, Knoxville, TN 37849 (US); MCKNIGHT, Mark E., Knoxville, TN 37924 (US); PATRICK, Sallie P, Maryville, TN 37803 (US); JONES, Kenneth L., Lenoir City, TN 37772 (US); TUCKER, James W., Knoxville, TN 37932 (US); RUSEV, Vladimir, Cumming, GA 30041 (US)
(74) Representative: Walther Bayer Faber Patentanwälte PartGmbB

(57) **Abstract**

A system provides for tracking expiration dates of individual inventory items in a healthcare facility and recommending actions for facility personnel to take to reduce risks to the healthcare facility and its patients caused by expired inventory items. The system determines whether individual inventory items are present within an inventory storage space. If an individual inventory item is present, it is managed to reduce the likelihood that its expiration date will arrive while it is still in the inventory storage space. The recommended actions include (1) returning the item to the vendor, (2) moving the item to a position in the storage space where it is most accessible to be selected for use, (3) relocating the item to another department within the healthcare facility where demand for the item is greater, and (4) shipping the item to another healthcare facility where demand for the item is greater.

## Description

### FIELD

This invention relates to the use of radio frequency identification (RFID) technology to identify products within an inventory space and access information about the identified products. More particularly, this invention relates to use of RFID technology to determine appropriate actions to take with respect to products having approaching expiration dates and directing responsible personnel to take such actions.

### BACKGROUND

Many healthcare systems have multiple facilities at multiple locations, each with multiple departments. Generally, each department at each location keeps an inventory of products needed for patient care. Due to various factors, different facilities and departments may utilize the same product at different rates. Factors affecting product utilization rates include physician preference, patient population, and procedure volume, among others.

A hospital department (e.g., Cath Lab, Interventional Radiology, Surgery, etc.) may have hundreds or thousands of types of products that hospital staff must manage. For some products that are used infrequently, the hospital department may keep only one on the shelf. For other products that are used more frequently, the hospital department may stock several of each product on the shelf. Considering the number of different types of products used in each department, and multiples of many of the products, a hospital may have tens of thousands of individual products that must be managed.

Most products used in procedure areas of a hospital have expiration dates. Manually tracking the expiration dates of thousands or tens of thousands of products requires an enormous amount of effort. As a result, many hospitals end up with products reaching their expiration dates while sitting on the shelf. These expired products represent both a cost to the hospital and a risk to patients. Ideally, expired products are quickly identified and disposed of, and the hospital orders replacements. If expired products are left on the shelf, the expired product could accidentally be used during a patient's procedure. The cost to a hospital of expired products could easily total hundreds of thousands of dollars per year.

What is needed, therefore, is a system for tracking expiration dates of individual products at a healthcare facility, and for recommending actions to avoid expiration of products on the shelves of the facility, thereby reducing patient risk and minimizing financial losses for the healthcare facility.

### SUMMARY

The above and other needs are met by an apparatus and method that tracks expiration dates of individual inventory items in a healthcare facility and recommends actions to reduce risks to the healthcare facility and its patients. In preferred embodiments, the system determines whether or not individual inventory items are on a shelf in an inventory storage space. If an individual inventory item is not on the shelf, it is assumed to have been consumed in patient care. If an individual inventory item is on the shelf, it is managed to reduce the likelihood that its expiration date will arrive while it is still on the shelf.

It will be appreciated that some product vendors provide contractual terms to hospitals and other healthcare facilities that allow for products to be returned to the vendor for a replacement, so long as the products are not past a certain time relative to their expiration dates. For example, such contractual terms may allow for a product to be returned so long as it is not within a certain number of days (such as 30 or 60) of its expiration date. Some more lenient contractual terms may allow for return of a product as long as it has not reached the expiration date.

Some embodiments described herein provide instructions or recommendations regarding actions for healthcare facility personnel to take as the expiration dates of inventory items on the shelves are approaching. These actions should reduce the likelihood that an individual inventory item will still be on the shelf at a healthcare facility on its expiration date. Some possible actions include:
- returning an individual inventory item to the vendor;
- moving an individual inventory item to a front position on the shelf where it is most accessible to be selected for use;
- relocating an individual inventory item to another department within the healthcare facility where demand for the item is greater; and
- shipping an individual inventory item to another healthcare facility where demand for the item is greater.

In some embodiments, a progression of different actions will be recommended as the expiration date approaches. For example:
- at 180 days before expiration, the recommended action may be to move the individual inventory item to a front position on the shelf;
- at 120 days before expiration, the recommended action may be to return the individual inventory item to the vendor; and
- at 90 days before expiration, if the individual inventory item has not been returned to the vendor, the recommended action may be to ship the item to another healthcare facility where demand for the item is greater.

One preferred embodiment described herein is directed to an apparatus for tracking expiration dates of inventory items and communicating recommendations for actions to take to reduce healthcare system risks caused by expiration of inventory items. The apparatus includes a first inventory space associated with a first healthcare facility, a second inventory space associated with the first healthcare facility or a second healthcare facility, an inventory database, and an inventory computer, all of which are in communication with each other via a communication network. The first inventory space includes individual inventory items, RFID tags, an RFID reader, and a user interface device. The individual inventory items are for use in medical treatment of patients. Each individual inventory item is associated with a corresponding inventory item code. Each RFID tag is attached to a corresponding one of the individual inventory items and is encoded with a tag identification number. A tag identification number could be a TID (Tag ID), EPC (Electronic Product Code), or any other locally or globally unique identifier tracked in the memory of the tag. The TID is a unique number assigned by the tag's chip manufacturer. Optionally, another memory bank of the tag, for example user memory, could be utilized to hold a unique identifier. This optional unique identifier could be locally unique to the customer or globally unique like the TID. The RFID reader is operable to scan the first inventory space to detect the RFID tags and acquire tag identification numbers therefrom. The user interface device is operable to receive and display item information and messages to facility personnel, wherein the item information and messages are related to the individual inventory items. The inventory database associates tag identification numbers with item information that includes inventory item codes and item expiration dates.

The inventory computer is operable to execute instructions to:
- receive a tag identification number from the RFID reader;
- acquire item information from the inventory database, wherein the item information is associated with the received tag identification number and includes an expiration date of the individual inventory item associated with the tag identification number;
- compare the expiration date to the current date;
- based on the difference between the current date and the expiration date, determine whether a timing rule is triggered;
- if no timing rule is triggered, generate no message that includes a recommendation to move the individual inventory item; and
- if a timing rule is triggered, generate a message that includes a recommendation to move the individual inventory item, and transmit the message via the communication network.

The user interface device is further operable to receive messages from the communication network and display the recommendations included therein to facility personnel.

In some embodiments, the inventory computer generates no message that includes a recommendation to move the individual inventory item if the current date is less than X1 days and greater than or equal to X2 days before the expiration date.

In some embodiments, if a first timing rule is triggered based on the current date being less than X1 days and greater than or equal to X2 days before the expiration date, the inventory computer generates a first message that includes a recommendation to move the individual inventory item to a position within the first inventory space that increases accessibility of the individual inventory item for selection for use in medical treatment of a patient.

In some embodiments, if a second timing rule is triggered based on the current date being less than X2 days and greater than or equal to X3 days before the expiration date, the inventory computer generates a second message that includes a recommendation to return the individual inventory item to the vendor of the individual inventory item.

In some embodiments, the inventory computer generates only the second message if the current date is less than X2 days before the expiration date.

In some embodiments, if a third timing rule is triggered based on the current date being less than X3 days and greater than or equal to X4 days before the expiration date, the inventory computer generates a third message that includes a recommendation to move the individual inventory item to the second inventory space, wherein the second inventory space is associated with the first healthcare facility.

In some embodiments, if a fourth timing rule is triggered based on the current date being less than X4 days before the expiration date, the inventory computer generates a fourth message that includes a recommendation to move the individual inventory item to the second inventory space, wherein the second inventory space is associated with the second healthcare facility.

In some embodiments, any particular message that includes a particular recommendation for any particular individual inventory item is generated only one time by the inventory computer for that particular individual inventory item.

In some embodiments, the inventory computer generates the messages based on timing rules. If no timing rule is triggered by the difference between the current date and the expiration date, no message is generated for the individual inventory item. If one or more timing rules are triggered by the difference between the current date and the expiration date, one or more corresponding messages are generated for the individual inventory item.

In some embodiments, the timing rules are based on the difference between the current date and the expiration date being equal to a value. For example, when the difference between the current date and the expiration date is 120 days, the inventory computer generates a message recommending that the individual inventory item be returned to its vendor.

In some embodiments, the timing rules are based on the difference between the current date and the expiration date being between two values. For example, when the difference between the current date and the expiration date is between 90 days and 120 days, the inventory computer generates a message recommending the individual inventory item be returned to its vendor.

In some embodiments, the timing rules are different for some of the inventory items. For example, for some of the items, the recommendation will be to return the individual inventory item to its vendor at 90 days before expiry, while for other items it will be at 120 days, and for still other items it will be at 150 days.

In some embodiments, the generated messages include one or more of the following: (1) move the individual inventory item to a position within the first inventory space that increases accessibility of the individual inventory item for selection for use in medical treatment of a patient; (2) return the individual inventory item to its vendor; (3) move the individual inventory item to the second inventory space, wherein the second inventory space is associated with the first healthcare facility; (4) move the individual inventory item to the second inventory space, wherein the second inventory space is associated with the second healthcare facility; or (5) contact the vendor to substitute the individual inventory item with another individual inventory item with a later expiration date.

In some embodiments, a user can create customized messages that are generated based on timing rules.

In some embodiments, the first inventory space includes an RFID association device that is connected to the communication network. The RFID association device is operable to encode each RFID tag with the inventory item code of the individual inventory item to which the RFID tag is attached. The RFID association device is also operable to communicate the inventory item codes and the associated tag identification numbers to the inventory database via the communication network.

In some embodiments, the communication network comprises the Internet and/or a local area network within the first healthcare facility.

In some embodiments, the first inventory space comprises a room, closet, vault, or cabinet in which the individual inventory items are stored prior to removal for use in medical treatment of patients. In some embodiments, the first inventory space includes inventory storage structures in which the individual inventory items are stored.

In some embodiments, the inventory computer generates the first message to include a recommendation to move the individual inventory item to a position on an inventory storage structure that increases accessibility of the individual inventory item for selection for use in medical treatment of a patient.

In some embodiments, the inventory storage structures comprise shelving units and/or bins on shelving units.

In some embodiments, the RFID reader comprises a handheld RFID scanner or a fixed RFID scanner installed within the first inventory space.

In some embodiments, the user interface device is a handheld device, such as a tablet computer, laptop computer, or smart phone that is in wireless communication with the communication network.

In some embodiments, if the current date is greater than X1 days, the inventory computer does not generate a message that includes a recommendation to move the individual inventory item
In some embodiments, the recommendations generated by the inventory computer are based at least in part on the cost of the individual inventory item or the cost to deliver or relocate the individual inventory item to the second inventory space.

In some embodiments, the recommendations generated by the inventory computer are based at least in part on the relative risk that the individual inventory item will reach its expiration date while in the second inventory space.

In another aspect, some embodiments of the invention provide a method for tracking expiration dates of inventory items and reducing healthcare system risks caused by expiration of inventory items. The method includes facility personnel using the apparatus described above to display the messages on the user interface device, and the facility personnel taking action according to the recommendations provided in the messages.

In yet another aspect, some embodiments of the invention provide a method for tracking expiration dates of inventory items and communicating recommendations for actions to take to reduce healthcare system risks caused by expiration of inventory items. The method includes:
- receiving individual inventory items into the first inventory storage space, wherein the individual inventory items are for use in medical treatment of patients, and each individual inventory item is associated with a corresponding inventory item code;
- attaching RFID tags to the individual inventory items, wherein each of the RFID tags is encoded with a tag identification number;
- encoding each of the RFID tags with the inventory item code of the individual inventory item to which the RFID tag is attached;
- associating tag identification numbers with item information in an inventory database, wherein the item information includes inventory item codes and item expiration dates;
- orienting the individual inventory items within the first inventory storage space to increase likelihoods that the RFID tags attached to the individual inventory items will be successfully read using an RFID reader disposed within the first inventory storage space;
- scanning the first inventory storage space using the RFID reader to detect the RFID tags attached to the individual inventory items;
- acquiring item information from the inventory database, wherein the item information is associated with the RFID tags detected within the first inventory storage space;
- executing instructions on an inventory computer that is in communication with the inventory database, wherein the instructions cause the inventory computer to:
   compare the current date to the expiration date of an individual inventory item for which item information was acquired from the inventory database;
   if the current date is less than X1 days and greater than or equal to X2 days before the expiration date, generate a first message that includes a recommendation to move the individual inventory item to a position within the first inventory space that increases the accessibility of the individual inventory item for selection for use in medical treatment of a patient;
   if the current date is less than X2 days and greater than or equal to X3 days before the expiration date, generate a second message that includes a recommendation to return the individual inventory item to its vendor;
   if the current date is less than X3 days and greater than or equal to X4 days before the expiration date, generate a third message that includes a recommendation to move the individual inventory item to the second inventory space, wherein the second inventory space is associated with the first healthcare facility;
   if the current date is less than X4 days before the expiration date, generate a fourth message that includes a recommendation to move the individual inventory item to the second inventory space, wherein the second inventory space is associated with the second healthcare facility; and
   transmit the first, second, third, and fourth messages via the communication network;
- displaying the recommendations included in the first, second, third, and fourth messages on a user interface device that is in communication with the communication network within the first healthcare facility; and
- facility personnel taking action according to the recommendations in the messages displayed on the user interface device.

### BRIEF DESCRIPTION OF THE DRAWINGS

Other embodiments of the invention will become apparent by reference to the detailed description in conjunction with the figures, wherein elements are not to scale so as to more clearly show the details, wherein like reference numbers indicate like elements throughout the several views, and wherein:
FIG. 1 depicts a product expiration management and risk mitigation system according to an embodiment of the invention;
FIG. 2 depicts components of a product expiration management and risk mitigation system disposed at an inventory space according to an embodiment of the invention; and
FIG. 3 depicts a method for managing product expiration and mitigating risk according to an embodiment of the invention.

### DETAILED DESCRIPTION

As the term is used herein, an "inventory item" or "product" is any item of inventory that is provided by a vendor and kept in inventory until the item is consumed or otherwise disposed of. In some embodiments described herein, an inventory item may be a "medical item," which is an item, device, material, substance, or piece of durable medical equipment (DME) that is kept in inventory in a medical facility until it is used or consumed during the performance of a medical procedure or dispensed to a patient to treat a medical condition or provide comfort to the patient. For example, sponges, gloves, and drapes are medical items. Surgical implants, knee braces, negative pressure wound therapy units, blood glucose monitors, and wheelchairs are further examples of medical items.

Each inventory item has a catalog number that may be referred to when placing an order for some quantity of the item. For example, a box of gloves has a catalog number. Inventory items are often referred to using SKUs (Stock Keeping Units). If someone says they have 1,000 SKUs, this means their inventory includes 1,000 different catalog numbers that identify 1,000 different types of inventory items. One unit of an inventory item, such as one box of gloves, is referred to herein as an individual inventory item or an individual product.

Each individual inventory item may also have a unique identifier, such as an Electronic Product Code (EPC), that is encoded in an RFID tag attached to the item or its packaging. In a preferred embodiment, the RFID tag is embedded in a single adhesive label that is attached to the item or its packaging. As the term is used herein, a "scan" for RFID tags refers to operations performed by an RFID reader to transmit signals and receive signals from RFID tags that are in range of the RFID reader and its associated antenna(s).

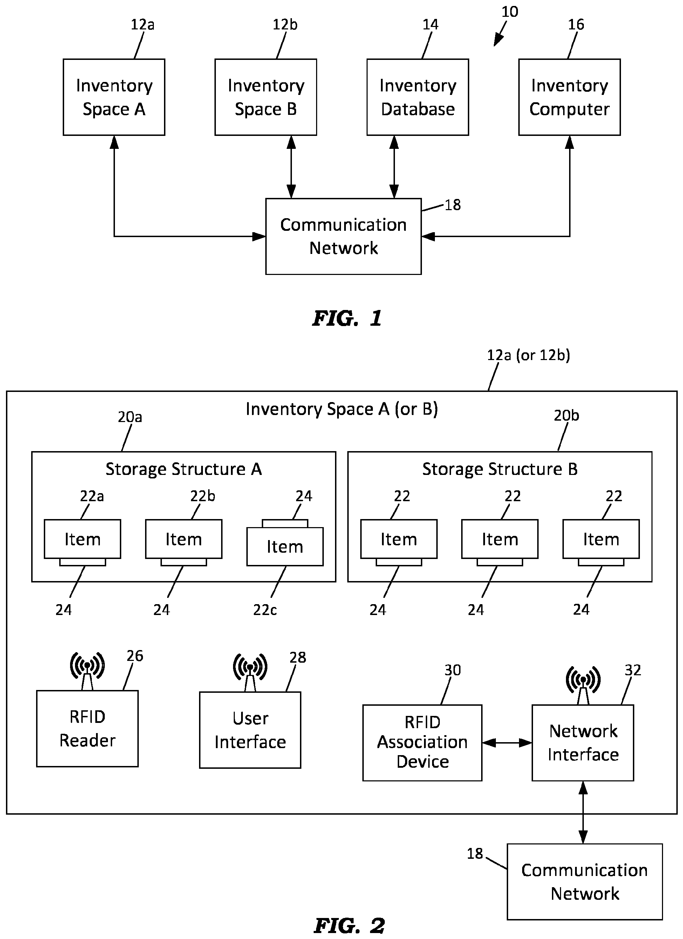

As shown in FIG. 1, a preferred embodiment of a product expiration management and risk mitigation system 10 includes a first inventory space 12a (also referred to as inventory space A), a second inventory space 12b (also referred to as inventory space B), an inventory database 14, a central inventory computer 16, and a communication network 18. The inventory spaces 12a-12b are storage spaces, such as rooms, closets, vaults, and cabinets, in which an inventory of items is stored until the items are removed. In one embodiment, the inventory spaces 12a-12b are separate rooms in which medical items are stored within one healthcare facility. For example, the inventory spaces 12a-12b may be in a Cath Lab department, an Interventional Radiology department, or a Surgery department. In another embodiment, the inventory spaces 12a-12b are in two separate healthcare facilities that may be in different regions of the country.

The inventory database 14 contains information regarding individual inventory item, such as information that associates the tag identification number of an individual inventory item with its SKU, information indicating the expiration date of the individual inventory item, and information identifying an inventory space in which the individual inventory item is stored.

The central inventory computer 16 may be a server computer, desktop computer, laptop computer, or other computing device. The inventory computer 16 includes memory for storing and a processor for executing instructions to communicate with the inventory database 14 to update and maintain inventory records, and communicate with facility personnel as described in more detail herein.

The communication network 18 may comprise a wide area network, such as the Internet, and/or a local area network within a particular healthcare facility. In the preferred embodiment, the first and second inventory spaces 12a-12b, the inventory database 14, and the central inventory computer 16 all include network interface devices that allow them to communicate with each other via the communication network 18.

FIG. 2 depicts components of a preferred embodiment of the first inventory space 12a. It will be appreciated that the second inventory space 12b may include the same or similar components, although it will not have the same inventory of individual inventory items as the first inventory space 12a. As shown in FIG. 2, the first inventory space 12a includes one or more storage structures 20a-20b that each contain individual inventory items 22. In a preferred embodiment, the storage structures 20a-20b comprise shelving units on which the items 22 are placed. In some embodiments, the storage structures 20a-20b include bins in which the items 22 are contained, and the bins are placed on the shelving units. An RFID tag 24 is attached to each individual inventory item 22.

In a preferred embodiment, each inventory space 12a-12b includes an RFID reader 26, which may be a handheld RFID scanner such as the model RFD40 UHF RFID Premium manufactured by Zebra Technologies Corporation. In some embodiments, one or multiple RFID readers 26 may be carried around by facility personnel from one inventory space to another within the healthcare facility for use therein. Preferably, each RFID reader 26 is wirelessly connected to a network interface 32, such as via a WiFi link, and the network interface 32 is connected to the communication network 18. Each inventory space 12a-12b is preferably equipped with at least one mobile user interface device 28, which may be a tablet computer, laptop computer, or smart phone that is wirelessly connected to a network interface 32.

In a preferred embodiment, each inventory space 12a-12b also includes an RFID association device 30, such as the model 47940911 Continuum Encoding Station Utility manufactured by DeRoyal Industries Inc. The RFID association device 30 is used to encode each RFID tag 24 with the EPC of the individual inventory item 22 to which the tag 24 is or will be attached. The RFID association device 30 is also operable to communicate with the inventory database 14 via the network interface 32 and the communication network 18, so that the tag ID of the RFID tag 24 can be stored in the database 14 in association with the EPC of each individual inventory item 22 and other item information. In a second embodiment, a unique tag identification number is stored in the tag's user memory. In a third embodiment, the tag's Tag ID is read from the tag and used as the tag identification number.

It will be appreciated that care must be taken to properly attach each RFID tag 24 to its associated individual inventory item 22 in a manner that will maximize the probability that the tag 24 will be readable by the RFID reader 26 when the tagged item is disposed within the storage structure 20a-20b. This involves choosing a surface of the item 22 or its packaging that is most likely to be in a readable orientation with respect to the RFID reader 26 when multiple items 22 are arranged together within the storage structure 20a-20b. For example, even though the RFID reader 26 is not an optical device, there is a higher probability that the reader 26 will be able to accurately read an RFID tag 24 if there is a clear line-of-sight between the reader 26 and the tag. As an illustration of this point, the two items 22a and 22b on the left side of the storage structure 20a in FIG. 2 are properly oriented with their RFID tags 24 facing outward with a clear line-of-sight to the reader 26. In contrast, the tag 24 on the item 22c on far right side of the storage structure 20a does not have a clear line-of-sight to the reader 26. As a result, there is a lower probability that the tag 24 on the item 22c will be successfully read. Another consideration is the choice of an RFID tag having a size and shape that is compatible with the size and shape of the surface of the item 22 that will have a clear line-of-sight to the reader 26 when the item 22 is disposed on the storage structure 20a.

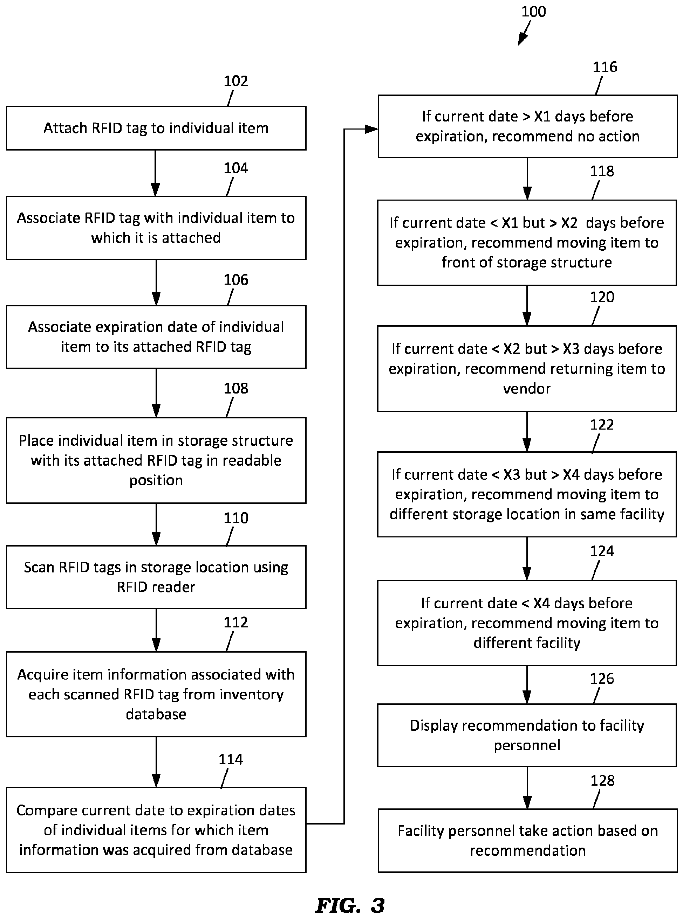

FIG. 3 depicts steps of a method 100 that may be carried out using the system shown in FIGS. 1 and 2 to manage product expiration and mitigate risk. The method 100 includes facility personnel attaching individual RFID tags 24 to the individual inventory items 22 or their packaging (step 102). As discussed above, the selection of the surfaces to which the tags 24 will be attached and the selection of the size and configuration of the tags 24 can affect the successful reading of the tags 24 by the RFID tag reader 26. The RFID association device 30 is used to encode each RFID tag 24 with an EPC, and store the EPC in the inventory database 14 in association with information about the individual inventory item 22 to which the tag 24 is attached (step 104). In some embodiments, this may involve reading the Tag ID of the tag 24 and storing the Tag ID in the inventory database 26 in association with other information about the individual inventory item 22. In some embodiments, step 104 may be performed before or after step 102. In step 106, information indicating the expiration date of the individual inventory item 22 is associated in the inventory database 14 with the Tag ID, the EPC, or both. Once the item 22 has been tagged, it is placed in the storage structure 20a or 20b with its RFID tag 24 in a proper position for successful reading (step 108). In another embodiment, a unique tag identification number is stored in the tag's user memory instead of the Tag ID or EPC.

From time to time, facility personnel will use the RFID tag reader 26 to scan the inventory of items in each inventory space 12a-12b (step 110). For each RFID tag 24 detected during a scan, the inventory computer 16 accesses the inventory database 14 to acquire item information associated with each detected RFID tag 24 (step 112). In preferred embodiments, the item information includes expiration dates of individual inventory items 22. For each tag 24 detected in the inventory space 12a-12b, the inventory computer 16 compares the item expiration date to the current date (which is preferably the date the tag 24 is detected), and determines how many days remain until the expiration date (step 114).

In preferred embodiments, the system 10 generates a recommendation for action that facility personnel should take with regard to each individual inventory item 22 detected within the inventory space 12a-12b, wherein the recommendation is based at least in part on the number days remaining until the expiration date of the individual inventory item 22. For example:
- If there are more than X1 days until expiration, the recommendation may be to take no immediate action, so that the individual inventory item 22 remains in its current position within the inventory space 12a-12b (step 116).
- If there are fewer than X 1 days but greater than X2 days until expiration, the recommended action may be to move the individual inventory item 22 to a front position, or top position, or otherwise most assessable position on the storage structure 20a-20b (step 118).
- If there are fewer than X2 days but greater than X3 days until expiration, the recommended action may be to return the individual inventory item 22 to the product's vendor for replacement or refund (step 120).
- If there are fewer than x3 but greater than X4 days until expiration (which means that at least some of the previously recommended actions were either not taken or not needed), the recommended action may be to move the individual inventory item 22 to another inventory space 12a-12b within the same healthcare facility where overall demand for the item is greater (step 122).
- If there are fewer than X4 days until expiration (which means that at least some of the previously recommended actions were either not taken or not needed), the recommended action may be to ship the individual inventory item 22 to another healthcare facility where overall demand for the item is greater (step 124).
In some embodiments, X1 = 180, X2 = 120, X3 = 90, and X4 = 60.
In some embodiments, healthcare systems may skip some of the generated action steps. For example:
- Some healthcare systems may elect to not move soon-to-expire items to a front position, or top position, or otherwise most assessable position on the storage structure.
- Some healthcare systems may elect to focus efforts towards returning products to vendors for replacement or refund. In these embodiments the only recommended actions may be to return products to vendors.
- Some healthcare systems may elect to vary the recommended actions based on the inventory item. For example, all possible actions may be recommended for some inventory items, no actions may be recommended for some inventory items, only one action may be recommended for some inventory items, only two actions may be recommended for some inventory items, etc.

In preferred embodiments, the recommendation for action is included in an electronic message that is transmitted to and displayed on the user interface device 28 (step 126), which prompts facility personnel to take the recommended action (step 128).

In some embodiments, the RFID reader 26 is a fixed device that is installed within the inventory space 12a-12b, and it is automatically activated periodically to scan for RFID tags 24. In this case, the inventory computer 16 compares the current date to item expiration dates associated with the detected tags, determines how many days remain until the associated individual items expire, and generates recommendations for action accordingly. The action recommendations may be provided to facility personnel via email or other messaging application.

Thus, in preferred embodiments, the inventory computer 16 determines a recommended action based at least in part on timing rules regarding when to take each action. In some embodiments, the inventory computer 16 may also determine a recommended action based at least in part on the cost of the individual inventory item 22, the cost to deliver or relocate the individual inventory item 22, and the relative risk of the individual inventory item 22 expiring in another inventory space 12a-12b or another healthcare facility.

The foregoing description of preferred embodiments for this invention have been presented for purposes of illustration and description. They are not intended to be exhaustive or to limit the invention to the precise form disclosed. Obvious modifications or variations are possible in light of the above teachings. The embodiments are chosen and described in an effort to provide the best illustrations of the principles of the invention and its practical application, and to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally, and equitably entitled.

## Claims

1. An apparatus for tracking expiration dates of inventory items and communicating recommendations for actions to take to reduce healthcare system risks caused by expiration of inventory items, the apparatus comprising:
a communication network for transferring item information regarding the inventory items;
a first inventory space associated with a first healthcare facility, the first inventory space comprising:
a plurality of individual inventory items for use in medical treatment of patients, each individual inventory item associated with a corresponding inventory item code;
a plurality of RFID tags, each attached to a corresponding one of the plurality of individual inventory items, and each RFID tag encoded with a tag identification number;
an RFID reader that is operable to scan the first inventory space to detect one or more of the plurality of RFID tags and acquire one or more tag identification numbers therefrom, the RFID reader in communication with the communication network; and
a user interface device that is operable to receive and display one or more of item information and messages to facility personnel, the item information and messages related to one or more of the individual inventory items, wherein the user interface device is in communication with the communication network; and
a second inventory space associated with the first healthcare facility or a second healthcare facility;
an inventory database that associates tag identification numbers with item information, wherein the item information includes inventory item codes and item expiration dates, wherein the inventory database is in communication with the communication network; and
an inventory computer in communication with the inventory database, the RFID reader, and the user interface device via the communication network, the inventory computer operable to execute instructions to:
- receive a tag identification number from the RFID reader;
- acquire from the inventory database item information associated with the received tag identification number, wherein the item information includes an expiration date of the individual inventory item associated with the tag identification number;
- compare the expiration date to a current date;
- based on a difference between the current date and the expiration date, determine whether a timing rule is triggered;
- if no timing rule is triggered, generate no message that includes a recommendation to move the individual inventory item; and
- if a timing rule is triggered, generate a message that includes a recommendation to move the individual inventory item, and transmit the message via the communication network; and
the user interface device further operable to receive the message from the communication network and display the recommendations included therein to facility personnel.

2. The apparatus of Claim 1 wherein the inventory computer generates no message that includes a recommendation to move the individual inventory item if the current date is less than X1 days and greater than or equal to X2 days before the expiration date.

3. The apparatus of Claim 1 wherein if a first timing rule is triggered based on the current date being less than X1 days and greater than or equal to X2 days before the expiration date, the inventory computer generates a first message that includes a recommendation to move the individual inventory item to a position within the first inventory space that increases accessibility of the individual inventory item for selection for use in medical treatment of a patient.

4. The apparatus of Claim 1 wherein if a second timing rule is triggered based on the current date being less than X2 days and greater than or equal to X3 days before the expiration date, the inventory computer generates a second message that includes a recommendation to return the individual inventory item to the vendor of the individual inventory item.

5. The apparatus of Claim 4 wherein the inventory computer generates only the second message if the current date is less than X2 days before the expiration date.

6. The apparatus of Claim 1 wherein if a third timing rule is triggered based on the current date being less than X3 days and greater than or equal to X4 days before the expiration date, the inventory computer generates a third message that includes a recommendation to move the individual inventory item to the second inventory space, wherein the second inventory space is associated with the first healthcare facility.

7. The apparatus of Claim 1 wherein if a fourth timing rule is triggered based on the current date being less than X4 days before the expiration date, the inventory computer generates a fourth message that includes a recommendation to move the individual inventory item to the second inventory space, wherein the second inventory space is associated with the second healthcare facility.

8. The apparatus of Claim 1 wherein any particular message that includes a particular recommendation for any particular individual inventory item is generated only one time by the inventory computer for that particular individual inventory item.

9. The apparatus of Claim 1 wherein the first inventory space further comprises an RFID association device in communication with the communication network, the RFID association device operable to encode each of the plurality of RFID tags with the inventory item code of the individual inventory item to which the RFID tag is attached, and operable to communicate the inventory item codes and associated tag identification numbers to the inventory database via the communication network.

10. The apparatus of Claim 1 wherein the communication network comprises one or both of the Internet and a local area network within the first healthcare facility.

11. The apparatus of Claim 1 wherein the first inventory space comprises one or more of a room, closet, vault, and cabinet in which the plurality of individual inventory items are stored prior to removal therefrom for use in medical treatment of patients.

12. The apparatus of Claim 1 wherein the first inventory space comprises one or more inventory storage structures on which or in which the plurality of individual inventory items are stored.

13. The apparatus of Claim 12 wherein the inventory computer generates the first message that includes a recommendation to move the individual inventory item to a position within or on one of the inventory storage structures that increases accessibility of the individual inventory item for selection for use in medical treatment of a patient.

14. The apparatus of Claim 12 wherein the one or more inventory storage structures comprise one or more shelving units or one or more bins on one or more shelving units.

15. The apparatus of Claim 1 wherein the RFID reader comprises a handheld RFID scanner or a fixed RFID scanner installed within the first inventory space.

16. The apparatus of Claim 1 wherein the user interface device is a handheld device comprising a tablet computer, laptop computer, or smart phone that is in wireless communication with the communication network.

17. The apparatus of Claim 1 wherein one or more recommendations generated by the inventory computer are based at least in part on one or both of a cost of the individual inventory item and a cost to deliver or relocate the individual inventory item to the second inventory space.

18. The apparatus of Claim 1 wherein one or more recommendations generated by the inventory computer are based at least in part on a relative risk that the individual inventory item will reach its expiration date while in the second inventory space.

19. The apparatus of Claim 1 wherein the recommendation to move the individual inventory item is a recommendation to change the arrangement of the individual inventory item in the first inventory space.

20. The apparatus of Claim 1 wherein the recommendation to move the individual inventory item is a recommendation to move the individual inventory item to the second inventory space associated with the first healthcare facility or the second healthcare facility.

21. The apparatus of Claim 1 wherein the recommendation to move the individual inventory item is a recommendation to remove the individual inventory item from the first inventory space and return the individual inventory item to a vendor.

22. The apparatus of Claim 1 wherein the recommendation to move the individual inventory item is a recommendation to remove the individual inventory item from the first inventory space and dispose of the individual inventory item.

23. A method for tracking expiration dates of inventory items and reducing healthcare system risks caused by expiration of inventory items, the method comprising:
- facility personnel using the apparatus of Claim 1 to display one or more messages on the user interface device; and
- the facility personnel taking action according to one or more of the recommendations provided in the one or more messages.

24. A method for tracking expiration dates of inventory items and communicating recommendations for actions to take to reduce healthcare system risks caused by expiration of inventory items, the method comprising:
- receiving a plurality of individual inventory items into a first inventory storage space, the individual inventory items for use in medical treatment of patients, each individual inventory item associated with a corresponding inventory item code;
- attaching RFID tags to the individual inventory items, wherein each of the RFID tags is encoded with a tag identification number;
- acquiring a tag identification number for each of the RFID tags attached to the individual inventory items;
- associating the tag identification numbers with item information in an inventory database, wherein the item information includes inventory item codes and item expiration dates;
- orienting the individual inventory items within the first inventory storage space to increase likelihoods that the RFID tags attached to the individual inventory items will be successfully read using an RFID reader disposed within the first inventory storage space;
- scanning the first inventory storage space using the RFID reader to detect the RFID tags attached to the individual inventory items;
- acquiring item information from the inventory database, the item information associated with the RFID tags detected within the first inventory storage space;
- executing instructions on an inventory computer in communication with the inventory database, the instructions causing the inventory computer to:
compare a current date to the expiration date of one or more of the individual inventory items for which item information was acquired from the inventory database;
if the current date is less than X1 days and greater than or equal to X2 days before the expiration date, generate a first message that includes a recommendation to move the individual inventory item to a position within the first inventory space that increases accessibility of the individual inventory item for selection for use in medical treatment of a patient;
if the current date is less than X2 days and greater than or equal to X3 days before the expiration date, generate a second message that includes a recommendation to return the individual inventory item to the vendor of the individual inventory item;
if the current date is less than X3 days and greater than or equal to X4 days before the expiration date, generate a third message that includes a recommendation to move the individual inventory item to the second inventory space, wherein the second inventory space is associated with the first healthcare facility;
if the current date is less than X4 days before the expiration date, generate a fourth message that includes a recommendation to move the individual inventory item to the second inventory space, wherein the second inventory space is associated with the second healthcare facility; and
transmit one or more of the first, second, third, and fourth messages via the communication network;
- displaying one or more of the recommendations included in one or more of the first, second, third, and fourth messages on a user interface device that is in communication with the communication network within the first healthcare facility; and
- facility personnel taking action according to one or more of the recommendations in one or more of the first, second, third, and fourth messages displayed on the user interface device.

25. The method of Claim 24 wherein the first inventory space comprises one or more of a room, closet, vault, and cabinet in which the plurality of individual inventory items are stored prior to removal therefrom for use in medical treatment of patients.

26. The method of Claim 24 wherein the first inventory space comprises one or more inventory storage structures on which or in which the plurality of individual inventory items are stored.

27. The method of Claim 26 wherein the inventory computer generates the first message that includes a recommendation to move the individual inventory item to a position within or on one of the inventory storage structures that increases accessibility of the individual inventory item for selection for use in medical treatment of a patient.

28. The method of Claim 24 wherein the inventory computer generates no message that includes a recommendation to move the individual inventory item if the current date is greater than X1 days.

29. The method of Claim 24 wherein the inventory computer generates one or more of the recommendations based at least in part on one or both of a cost of the individual inventory item and a cost to deliver or relocate the individual inventory item to the second inventory space.

30. The method of Claim 24 wherein the inventory computer generates one or more of the recommendations based at least in part on a relative risk of the individual inventory item will reach its expiration date while in the second inventory space.
